# EUROPEAN PATENT APPLICATION

(11) **EP 4 265 623 A1**
(43) Date of publication of application: **25.10.2023**
(21) Application number: 21906592.7
(22) Date of filing: 14.12.2021
(51) Int. Cl.: C07F 5/02, C08F 10/00, C08F 4/6592

(54) **NOVEL BORATE COMPOUND**

(30) Priority: 17.12.2020 JP 2020209071
(71) Applicant: AGC INC., Chiyoda-ku, Tokyo 1008405 (JP)
(72) Inventor: FUJIMOTO, Takuya, Tokyo 100-8405 (JP)
(74) Representative: Müller-Boré & Partner Patentanwälte PartG mbB
(86) International application number: PCT/JP2021/045926
(87) International publication number: WO 2022/131230

(57) **Abstract**

The present invention aims to provide a borate compound useful as a cocatalyst for the polymerization of olefin and diene. The present invention relates to a borate compound represented by the following formula (1): 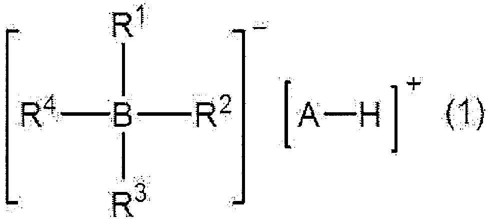 wherein each symbol is as defined in [DESCRIPTION], and use thereof as a cocatalyst for the polymerization of olefin and diene.

## Description

### [Technical Field]

The present invention relates to a borate compound useful as a cocatalyst for the polymerization of olefin and diene, and a production method thereof.

### [Background Art]

Many reports have been conventionally made on the use of metallocene compound and non-metallocene type metal complex catalysts such as diimine complex, phenoxy complex, and the like as catalysts for the polymerization of olefins and dienes. As cocatalysts used for stabilizing the cationic active species of these metal complex catalysts, aluminoxanes such as alkylaluminum, methylaluminoxane (MAO) and the like, Broensted acid salts such as ammonium borate and the like, and Lewis acid salts such as triphenylcarbenium borate and the like are used (Non-Patent Document 1).

In the catalyst activation reaction by the aforementioned Broensted acid salt, the leaving group on the metal complex catalyst is protonated and eliminated from the metal complex catalyst to generate a cationic active species of the metal complex catalyst, whereby non-coordinating anions derived from Broensted acid salt stabilize the active species. As the Broensted base constituting the Broensted acid salt, various borate compounds such as tetrakis(pentafluorophenyl)borate, which is a non-coordinating anion, and the like have been reported (Non-Patent Document 1) and, as the Broensted acid, Broensted acids containing nitrogen, phosphorus, oxygen, and/or sulfur are known (Patent Document 1).

As the aforementioned Broensted acid salts, Broensted acid salts (ammonium borates) containing nitrogen such as dimethylanilinium tetrakis(pentafluorophenyl)borate, tri(n-butyl)ammonium tetrakis(pentafluorophenyl)borate, methylpyrrolidinium tetrakis(pentafluorophenyl)borate, and the like are known (Patent Document 2). In the catalyst activation reactions by these ammonium borates, amine compounds are generated due to the loss of proton in the protonation stage. Such amine compound may interact with the cationic active species of the metal complex catalyst, in which case the compound is feared to adversely affect the polymerization reaction.

In addition, as a solvent used for polymerization, a nonpolar hydrocarbon solvent is used. In particular, from the aspects of odor and toxicity, switching to aliphatic hydrocarbon solvents such as n-hexane and the like from aromatic hydrocarbon solvents such as toluene and the like is progressing.

However, it is known that general tetrakis(pentafluorophenyl)borate compounds are hardly soluble in aromatic hydrocarbon solvents such as toluene and the like, and that even if dissolved, they are separated to form two liquid-liquid phases of a concentrated phase in which the borate compound is dissolved and a dilute phase in which it is not dissolved (Patent document 3).

In addition, since tetrakis(pentafluorophenyl)borate compounds are hardly soluble in aliphatic hydrocarbon solvents such as n-hexane, n-heptane, and the like, a compound soluble in aliphatic hydrocarbon solvents is desired and has been proposed (Patent document 4). Di(octadecyl)methylammonium tetrakis(pentafluorophenyl)borate and bis(hydrogenated tallow)methylammonium tetrakis(pentafluorophenyl)borate described in Patent document 4 are useful as compounds soluble in hydrocarbon solvents.

However, when di(octadecyl)methylammonium tetrakis(pentafluorophenyl)borate or bis(hydrogenated beef tallow alkyl)methylammonium tetrakis(pentafluorophenyl)borate described in Patent Document 4 is used, since trialkylamine generated after a catalyst activation reaction has nucleophilicity, it is feared that it becomes a catalyst poison in the polymerization reaction of olefins or dienes.

### [prior art document]

### [Non-Patent Document]

Non-Patent Document 1: Chem. Rev. 2000, 100, 1391-1434

### [Patent Document]

Patent Document 1: US Patent No. 5132380
Patent Document 2: WO 2010/014344
Patent Document 3: JP-A-2018-104335
Patent Document 4: Japanese Translation of PCT Application Publication No. 2000-507157

### [Summary of Invention]

### [Technical Problem]

In view of those conventional techniques, the present invention aims to provide a borate compound, which is soluble in hydrocarbon solvents and useful as a cocatalyst for the polymerization reaction of olefins and dienes, and an industrial production method thereof.

### [Solution to Problem]

The present inventors have conducted intensive studies and found for the first time that a compound represented by the following formula (1): 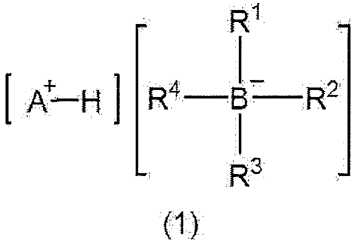
wherein R¹, R², R³ and R⁴ are each independently a C₆₋₁₄ aryl group substituted by one or more fluorine atoms or fluoro C₁₋₄ alkyl groups, and
[A⁺-H] is a cation in which the ring nitrogen atom of a 5- or 6-membered monocyclic nitrogen-containing aromatic heterocyclic compound having a total carbon number of not less than 25 and substituted by the same or different, two or more C₁₋₃₀ alkyl groups or C₁₋₃₀ alkoxy groups is protonated (hereinafter to be also referred to as "the compound of the present invention") does not allow generation of a compound to be a catalyst poison for the polymerization reaction of olefin and diene, and is useful as a cocatalyst for the polymerization reaction of olefin and diene, and completed the present invention.

Accordingly, the present invention provides the following.
[1] A compound represented by the following formula (1): 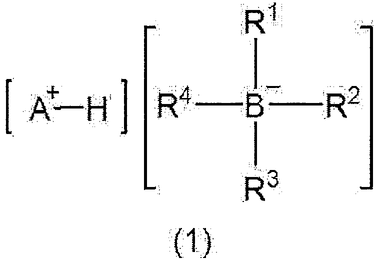
   wherein R¹, R², R³ and R⁴ are each independently a C₆₋₁₄ aryl group substituted by one or more fluorine atoms or fluoro C₁₋₄ alkyl groups, and
   [A⁺-H] is a cation in which the ring nitrogen atom of a 5- or 6-membered monocyclic nitrogen-containing aromatic heterocyclic compound having a total carbon number of not less than 25 and substituted by the same or different, two or more C₁₋₃₀ alkyl groups or C₁₋₃₀ alkoxy groups is protonated.
[2] The compound of the aforementioned [1], wherein R¹, R², R³ and R⁴ are each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 9-phenanthryl group, or a 3-phenanthryl group, each of which is substituted by one or more fluorine atoms or trifluoromethyl groups.
[3] The compound of the aforementioned [1], wherein all of R¹, R², R³ and R⁴ are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups.
[4] The compound of any of the aforementioned [1] to [3], wherein
   A is a 5- or 6-membered monocyclic bicyclic nitrogen-containing aromatic heterocyclic compound having a total carbon number of not less than 25 and substituted by the same or different two C₉₋₃₀ alkyl groups or C₉₋₃₀ alkoxy groups.
[5] The compound of the aforementioned [4], wherein the 5- or 6-membered monocyclic nitrogen-containing aromatic heterocyclic compound is pyridine or imidazole.
[6] A cocatalyst for polymerization of at least one kind of monomer selected from the group consisting of an olefin and a diene, consisting of the compound of any of the aforementioned [1] to [5] .
[7] A method for producing a polymer, comprising polymerizing at least one kind of monomer selected from the group consisting of an olefin and a diene by using the compound of any of the aforementioned [1] to [5] as a cocatalyst.

### [Advantageous Effects of Invention]

According to the present invention, the aforementioned borate compound, which is soluble in hydrocarbon solvents and useful as a cocatalyst for the polymerization reaction of olefins and dienes can be provided.

### [Description of Embodiments]

The definitions of the terms and respective symbols used in the present specification are explained below.

In the present specification, the "halogen atom" means a fluorine atom, a chlorine atom, a bromine atom, or an iodine atom.

In the present specification, the "alkyl (group)" means a linear or branched chain alkyl group having a carbon atom number of not less than 1.

In the present specification, the "C₁₋₃₀ alkyl (group)" means a linear or branched chain alkyl group having a carbon atom number of 1 to 30. Examples thereof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl, tridecyl, tetradecyl, hexadecyl, octadecyl, nonadecyl, eicosyl, docosyl, tricosyl, tetracosyl, pentacosyl, hexacosyl, heptacosyl, octacosyl, nonacosyl, triacontyl, and the like.

In the present specification, the "C₉₋₃₀ alkyl (group)" means a linear or branched chain alkyl group having a carbon atom number of 9 to 30. Examples thereof include nonyl, decyl, undecyl, dodecyl, tridecyl, tetradecyl, hexadecyl, octadecyl, nonadecyl, eicosyl, docosyl, tricosyl, tetracosyl, pentacosyl, hexacosyl, heptacosyl, octacosyl, nonacosyl, triacontyl, and the like.

In the present specification, the "C₁₋₆ alkyl (group)" means a linear or branched chain alkyl group having a carbon atom number of 1 to 6. Examples thereof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, and the like. Among them, C₁₋₄ alkyl group is preferred.

In the present specification, the "halo C₁₋₆ alkyl (group)" means the aforementioned "C₁₋₆ alkyl" group in which one or more hydrogen atoms are substituted by halogen atom(s). Specific examples thereof include difluoromethyl, trifluoromethyl, 2-chloroethyl, 2-bromoethyl, 2-iodoethyl, 2-fluoroethyl, 2,2-difluoroethyl, 2,2,2-trifluoroethyl, pentafluoroethyl, 2,2,3,3-tetrafluoropropyl, 3,3,3-trifluoropropyl, 4,4,4-trifluorobutyl, 5,5,5-trifluoropentyl, 6,6,6-trifluorohexyl, and the like. Among them, "halo C₁₋₄ alkyl" is preferred.

In the present specification, the "fluoro C₁₋₆ alkyl (group)" means the aforementioned "halo C₁₋₆ alkyl" group in which the halogen atom is a fluorine atom. Specific examples thereof include difluoromethyl, trifluoromethyl, 2-fluoroethyl, 2,2-difluoroethyl, 2,2,2-trifluoroethyl, pentafluoroethyl, 2,2,3,3-tetrafluoropropyl, 3,3,3-trifluoropropyl, 4,4,4-trifluorobutyl, 5,5,5-trifluoropentyl, 6,6,6-trifluorohexyl, and the like. Among them, "fluoro C₁₋₄ alkyl (groups)" such as difluoromethyl, trifluoromethyl, 2,2,2-trifluoroethyl, pentafluoroethyl, 2,2,3,3-tetrafluoropropyl, 3,3,3-trifluoropropyl, 4,4,4-trifluorobutyl and the like are preferred, and difluoromethyl, trifluoromethyl, 2,2,2-trifluoroethyl, and pentafluoroethyl are more preferred, and trifluoromethyl is particularly preferred.

In the present specification, the "cycloalkyl (group)" means a cyclic alkyl group. Unless the carbon number range is particularly limited, it is preferably a C₃₋₈ cycloalkyl group.

In the present specification, the "C₃₋₈ cycloalkyl (group)" means a cyclic alkyl group having a carbon atom number of 3 to 8. Examples thereof include cyclopropyl, cyclobutyl, cyclopentyl, cyclohexyl, cycloheptyl, cyclooctyl, and the like. Among them, a C₃₋₆ cycloalkyl group is preferred.

In the present specification, the "alkoxy (group)" means a group in which a linear or branched chain alkyl group is bonded to an oxygen atom.

In the present specification, the "C₁₋₃₀ alkoxy (group)" means a linear or branched chain alkoxy group having a carbon atom number of 1 to 30. Examples thereof include methoxy, ethoxy, propoxy, isopropoxy, butoxy, isobutoxy, sec-butoxy, tert-butoxy, pentyloxy, isopentyloxy, neopentyloxy, hexyloxy, isohexyloxy, 1,1-dimethylbutoxy, 2,2-dimethylbutoxy, 3,3-dimethylbutoxy, 2-ethylbutoxy, heptyloxy, octyloxy, nonyloxy, decyloxy, undecyloxy, dodecyloxy, tridecyloxy, tetradecyloxy, hexadecyloxy, octadecyloxy, nonadecyloxy, eicosyloxy, docosyloxy, tricosyloxy, tetracosyloxy, pentacosyloxy, hexacosyloxy, heptacosyloxy, octacosyloxy, nonacosyloxy, triacontyloxy, and the like.

In the present specification, the "C₉₋₃₀ alkoxy (group)" means a linear or branched chain alkoxy group having a carbon atom number of 9 to 30. Examples thereof include nonyloxy, decyloxy, undecyloxy, dodecyloxy, tridecyloxy, tetradecyloxy, hexadecyloxy, octadecyloxy, nonadecyloxy, eicosyloxy, docosyloxy, tricosyloxy, tetracosyloxy, pentacosyloxy, hexacosyloxy, heptacosyloxy, octacosyloxy, nonacosyloxy, triacontyloxy, and the like.

In the present specification, the "C₁₋₆ alkoxy (group)" means a linear or branched chain alkoxy group having a carbon atom number of 1 to 6. Examples thereof include methoxy, ethoxy, propoxy, isopropoxy, butoxy, isobutoxy, sec-butoxy, tert-butoxy, pentyloxy, isopentyloxy, neopentyloxy, hexyloxy, and the like. Among them, a C₁₋₄ alkoxy group is preferred.

In the present specification, the "halo C₁₋₆ alkoxy (group)" means the aforementioned "C₁₋₆ alkoxy" group in which one or more hydrogen atoms are substituted by halogen atom(s). Specific examples thereof include difluoromethoxy, trifluoromethoxy, 2-chloroethoxy, 2-bromoethoxy, 2-iodoethoxy, 2-fluoroethoxy, 2,2-difluoroethoxy, 2,2,2-trifluoroethoxy, pentafluoroethoxy, 2,2,3,3,3-pentafluoropropoxy, 2,2,3,3-tetrafluoropropoxy, 3,3,3-trifluoropropoxy, 4,4,4-trifluorobutoxy, 5,5,5-trifluoropentyloxy, 6,6,6-trifluorohexyloxy, and the like. Among them, "halo C₁₋₄ alkoxy" is preferred.

In the present specification, the "fluoro C₁₋₆ alkoxy (group)" means the aforementioned "halo C₁₋₆ alkoxy" group in which the halogen atom is a fluorine atom. Specific examples thereof include difluoromethoxy, trifluoromethoxy, 2-fluoroethoxy, 2,2-difluoroethoxy, 2,2,2-trifluoroethoxy, pentafluoroethoxy, 2,2,3,3-tetrafluoropropoxy, 3,3,3-trifluoropropoxy, 4,4,4-trifluorobutoxy, 5,5,5-trifluoropentyloxy, 6,6,6-trifluorohexyloxy, and the like. Among them, "fluoro C₁₋₄ alkoxy (groups)" such as difluoromethoxy, trifluoromethoxy, 2,2,2-trifluoroethoxy, pentafluoroethoxy, 2,2,3,3,3-pentafluoropropoxy, 2,2,3,3-tetrafluoropropoxy, 3,3,3-trifluoropropoxy, 4,4,4-trifluorobutoxy, and the like are preferred; difluoromethoxy, trifluoromethoxy, 2,2,2-trifluoroethoxy, and pentafluoroethoxy are more preferred; and trifluoromethoxy is particularly preferred.

In the present specification, the "aryl (group)" mean a monocyclic or polycyclic (fused) hydrocarbon group showing aromaticity. Specific examples thereof include C₆₋₁₄ aryl groups such as phenyl, 1-naphthyl, 2-naphthyl, 2-biphenylyl, 3-biphenylyl, 4-biphenylyl, 1-anthryl, 2-anthryl, 9-anthryl, 3-phenanthryl, 9-phenanthryl, and the like. Among them, phenyl, 1-naphthyl, and 2-naphthyl are preferred.

In the present specification, the "5- or 6-membered monocyclic nitrogen-containing aromatic heterocyclic compound" means a 5- or 6-membered monocyclic aromatic heterocyclic compound containing, besides a carbon atom, 1 to 4 hetero atoms selected from a nitrogen atom, a sulfur atom, and an oxygen atom as ring-constituting atom(s), and containing at least one nitrogen atom as the ring-constituting atom.

Preferable examples of the "5- or 6-membered monocyclic nitrogen-containing aromatic heterocyclic compound" include pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine, and the like. Among them, pyridine and imidazole are more preferred.

In the present specification, the "optionally substituted" means unsubstituted or having one or more substituents. Unless otherwise particularly indicated, (1) a halogen atom, (2) a nitro group, (3) a cyano group, (4) a C₁₋₃₀ alkyl group, (5) a halo C₁₋₆ alkyl group, (6) a C₃₋₈ cycloalkyl group, (7) a C₁₋₃₀ alkoxy group, (8) a halo C₁₋₆ alkoxy group, (9) a C₆₋₁₄ aryl group, and the like can be mentioned as the "substituent". Among them, a halogen atom, a cyano group, a C₁₋₆ alkyl group, a halo C₁₋₆ alkyl group, a C₁₋₆ alkoxy group, a halo C₁₋₆ alkoxy group, and a phenyl group are preferred, and a halogen atom (e.g., fluorine atom), a C₁₋₆ alkyl group (e.g., methyl, ethyl), a C₁₋₆ alkoxy group (e.g., methoxy, ethoxy), and a halo C₁₋₆ alkyl group (e.g., trifluoromethyl) are more preferred. When plural substituents are present, respective substituents may be the same or different. The above-mentioned substituents may also be further substituted by one or more of a C₁₋₆ alkyl group, a C₁₋₆ alkoxy group, a halogen atom, a phenyl group, and the like.

In the present specification, the "hydrocarbon solvent" means solvents including aromatic hydrocarbon solvents and/or aliphatic hydrocarbon solvents. Among them, aliphatic hydrocarbon solvents are preferable from the aspects of odor and toxicity.

In the present specification, examples of the "aromatic hydrocarbon solvent" include benzene, toluene, xylene, and the like.

In the present specification, examples of the "aliphatic hydrocarbon solvent" include n-hexane, isohexane, heptane, octane, cyclohexane, methylcyclohexane, a mixed solvent thereof, and the like.

In the present specification, the "soluble in hydrocarbon solvent" means that the compound of the present invention is dissolved in a solution of a hydrocarbon solvent and the compound of the present invention at 25°C at a concentration of not less than 5 wt% to form a clear homogeneous solution.

### (Compound of the present invention)

The compound of the present invention is explained below.

The compound of the present invention is a compound represented by the following formula (1): 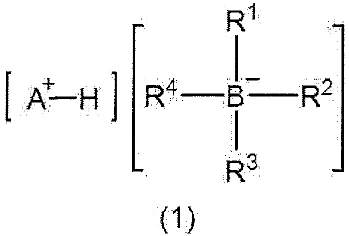
wherein R¹, R², R³ and R⁴ are each independently a C₆₋₁₄ aryl group substituted by one or more fluorine atoms or fluoro C₁₋₄ alkyl groups, and
[A⁺-H] is a cation in which the ring nitrogen atom of a 5- or 6-membered monocyclic nitrogen-containing aromatic heterocyclic compound having a total carbon number of not less than 25 and substituted by the same or different, two or more C₁₋₃₀ alkyl groups or C₁₋₃₀ alkoxy groups is protonated.

A preferred embodiment of A is explained below.

A is preferably a 5- or 6-membered monocyclic nitrogen-containing aromatic heterocyclic compound (e.g., pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine, etc.) substituted by the same or different two C₉₋₃₀ alkyl groups or C₉₋₃₀ alkoxy groups, more preferably pyridine or imidazole substituted by the same or different two C₁₄₋₃₀ alkyl groups or C₁₄₋₃₀ alkoxy groups.

A preferably has a total carbon number of not less than 25, more preferably has a total carbon number of not less than 30, further preferably not less than 35.

Specific preferable examples of A include 2,5-dinonadecylpyridine, 2,6-dinonadecylpyridine, 2-nonadecyl-5-octadecylpyridine, 2-nonadecyl-4-octadecyloxypyridine, 2-nonadecyl-6-octadecyloxypyridine, 4-nonadecyl-1-octadecylimidazole, 5-nonadecyl-1-octadecylimidazole, 2-nonadecyl-1-octadecylimidazole, and the like.

### (Production method of A (a 5- or 6-membered monocyclic nitrogen-containing aromatic heterocyclic compound having a total carbon number of not less than 25 and substituted by the same or different, two or more C₁₋₃₀ alkyl groups or C₁₋₃₀ alkoxy groups))

The aforementioned A can be produced by successively reacting, as shown in the following formula: 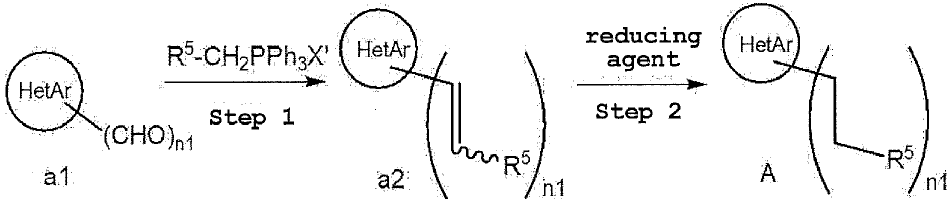 wherein the group represented by the formula: 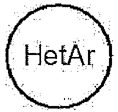 is a 5- or 6-membered monocyclic nitrogen-containing aromatic heterocyclic group, X' is a halogen atom, R⁵ is an optionally substituted C₁₋₃₀ alkyl group, and n1 is an integer of two or more, reacting compound (a1) with a phosphonium salt (R⁵-CH₂PPh₃X') in a solvent that does not effect the reaction in the presence of a base to give compound (a2) (step 1), and reacting the compound with a reducing agent (step 2).

Examples of the base to be used in the aforementioned step 1 include sodium hydride, potassium carbonate, potassium tert-butoxide, and the like.

The amount of the base to be used is 1 to 2 mol (preferably, 1 to 1.2 mol) with respect to the equivalent (1 mol) of formyl group of the compound (a1).

The amount of the phosphonium salt (R⁵-CH₂PPh₃X') to be used is 1 to 2 mol (preferably, 1 to 1.2 mol) with respect to the equivalent (1 mol) of formyl group of the compound (a1).

The reaction solvent in step 1 is not particularly limited and, for example, ether solvents such as tetrahydrofuran, diethoxy ethane, and the like, aromatic hydrocarbon solvents such as toluene and the like, aliphatic hydrocarbon solvents such as n-hexane and the like, dimethylformamide, dimethyl sulfoxide, and the like are preferred.

The reaction temperature in step 1 is preferably room temperature to 180°C.

The reaction time in step 1 is generally 0.5 hr to 48 hr.

In the aforementioned step 2, as the reducing agent, for example, in the presence of a metal catalyst, hydrogen, ammonium formate, ammonium chloride, or the like can be used. As the metal catalyst, transition metal catalysts such as Pd/C, Pt/C, and the like are preferred.

The amount of the metal catalyst to be used is 0.001 to 1.0 mol (preferably 0.01 to 0.5 mol) with respect to the equivalent (1 mol) of a double bond of the compound (a2).

While the reaction solvent in step 2 is not particularly limited, for example, n-hexane, toluene, tetrahydrofuran, ethanol, and the like are preferred, and a mixed solvent thereof may also be used.

For the reduction reaction in step 2, conditions such as normal pressure, moderate pressure, and the like can be appropriately selected according to the progress of the reaction.

The reaction temperature in step 2 is preferably room temperature to 180°C.

The reaction time in step 2 is generally 1 hr to 72 hr.

Preferred embodiments of a compound represented by the aforementioned formula (1) (hereinafter to be also referred to as "compound (1)") are explained below.

In the following, each group of compound (1) is explained.

R¹, R², R³, and R⁴ are preferably each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 3-phenanthryl group, or a 9-phenanthryl group, each substituted by one or more fluorine atoms or fluoro C₁₋₄ alkyl groups (e.g., trifluoromethyl groups), more preferably each independently a phenyl group, a 1-naphthyl group, or a 2-naphthyl group, each substituted by one or more fluorine atoms or trifluoromethyl groups, particularly preferably R¹, R², R³, and R⁴ are all the same and are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups.

A preferred embodiment of A in the [A-H]⁺ which is an A-derived cation is the same as the one mentioned above.

As preferred compound (1), the following compounds can be mentioned.

### [Compound (1-1)]

Compound (1) of the aforementioned formula (1), wherein
R¹, R², R³ and R⁴ are each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 3-phenanthryl group, or a 9-phenanthryl group, each substituted by one or more fluorine atoms or fluoro C₁₋₄ alkyl groups (e.g., trifluoromethyl groups),
A is a 5- or 6-membered monocyclic nitrogen-containing aromatic heterocyclic compound (e.g., pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine, etc.) substituted by the same or different, two or more C₉₋₃₀ alkyl groups or C₉₋₃₀ alkoxy groups, and the total carbon number is not less than 25 (preferably not less than 30).

### [Compound (1-2)]

Compound (1) of the aforementioned formula (1), wherein
R¹, R², R³ and R⁴ are each independently a phenyl group, a 1-naphthyl group, or a 2-naphthyl group, each substituted by one or more fluorine atoms or trifluoromethyl groups,
A is pyridine or imidazole, each substituted by the same or different two C₁₄₋₃₀ alkyl groups or C₁₄₋₃₀ alkoxy groups (preferably 2,5-dinonadecylpyridine, 2,6-dinonadecylpyridine, 2-nonadecyl-5-octadecylpyridine, 2-nonadecyl-4-octadecyloxypyridine, 2-nonadecyl-6-octadecyloxypyridine, 4-nonadecyl-1-octadecylimidazole, 5-nonadecyl-1-octadecylimidazole, or 2-nonadecyl-1-octadecylimidazole), and the total carbon number is not less than 35.

### [Compound (1-3)]

Compound (1) of the aforementioned formula (1), wherein
R¹, R², R³ and R⁴ are all the same and are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups (preferably, pentafluorophenyl groups),
A is pyridine substituted by the same or different two C₁₄₋₃₀ alkyl groups or C₁₄₋₃₀ alkoxy groups (preferably 2,5-dinonadecylpyridine, 2,6-dinonadecylpyridine, 2-nonadecyl-5-octadecylpyridine, 2-nonadecyl-4-octadecyloxypyridine, or 2-nonadecyl-6-octadecyloxypyridine), and
   the total carbon number is not less than 35.

Specific preferred examples of compound (1) include 2,6-dinonadecylpyridinium tetrakis(pentafluorophenyl)borate, 2-nonadecyl-5-octadecyloxypyridinium tetrakis(pentafluorophenyl)borate, 4-nonadecyl-1-octadecylimidazolium tetrakis(pentafluorophenyl)borate, 5-nonadecyl-1-octadecylimidazolium tetrakis(pentafluorophenyl)borate, 2-nonadecyl-1-octadecylimidazolium tetrakis(pentafluorophenyl)borate, and the like.

The compound of the present invention is soluble in a hydrocarbon solvent at room temperature (15 to 30°C). In addition, conventionally-known borate-type cocatalysts are insoluble in aliphatic hydrocarbon solvents such as n-hexane and the like. In contrast, the compound of the present invention shows solubility also in aliphatic hydrocarbon solvents. Therefore, it is useful as a cocatalyst in homogenous polymerization reactions of olefins and dienes.

### (Production method of the compound of the present invention)

The production method of the compound of the present invention (hereinafter to be also referred to as "the production method of the present invention") is explained below.

The compound of the present invention preferably does not contain a hydrogenated borate compound (e.g., hydrogenated tetrakis(pentafluorophenyl)borate) represented by the below-mentioned formula (3), or metal salts of the below-mentioned tetra-substituted borate compounds (e.g., lithium tetrakis(pentafluorophenyl)borate), which can form a complex with an ether compound having a total carbon number of not more than 7 and become a catalyst poison. In addition, the composition of the present invention preferably does not contain an ether compound having a total carbon number of not more than 7 which can be a catalyst poison. Not containing an ether compound having a total carbon number of not more than 7 means that an ether compound having a total carbon number of not more than 7 is not detected as a result of ¹H-NMR analysis.

The production method of the present invention characteristically includes a step of reacting a hydrogenated borate compound represented by the following formula (3): 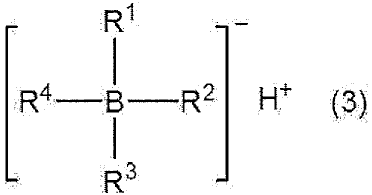 wherein R¹, R², R³, and R⁴ are as defined above (hereinafter to be also referred to as "compound (3)") with the aforementioned A, and uses A in an equimolecular amount (1 - 1.01 mol, preferably 1 mol) with respect to 1 mol of compound (3).

Examples of compound (3) to be used as a starting material in the present production method include known compounds such as hydrogenated tetrakis(pentafluorophenyl)borate, hydrogenated tetrakis(nonafluoro[1,1'-biphenyl]-4-yl)borate, hydrogenated tetrakis(heptafluoro-2-naphthyl)borate, hydrogenated [3,5-bis(trifluoromethyl)phenyl]borate, and the like.

The production method of compound (3) is not particularly limited and is, for example, a method including treating a compound represented by the formula (4): 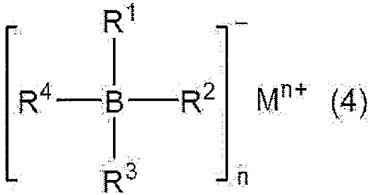
wherein R¹, R², R³, and R⁴ are each independently a C₆₋₁₄ aryl group substituted by one or more fluorine atoms or fluoro C₁₋₄ alkyl groups,
M is an alkali metal such as lithium, potassium, sodium, or the like, or an alkaline earth metal such as calcium, magnesium, barium, or the like, and
n is 1 or 2 (hereinafter to be also referred to as "compound (4)") with protonic acid, or the like.

As the aforementioned compound (4) used for the production of compound (3), a commercially available product or a purified product may be used, or one prepared by a method known per se (see, for example, Angew. Chem. Int. Ed., 2009, 48(40), 7444-7447) may also be used.

The solvent to be used in the production of compound (3) is not particularly limited, but it is desirable to use ether solvents such as diethyl ether, tert-butyl methyl ether, cyclopentyl methyl ether, diisopropyl ether, and the like, halogenated solvents such as dichloromethane, chloroform, and the like, aromatic hydrocarbon solvents such as toluene, benzene, and the like, and aliphatic hydrocarbon solvents such as n-hexane, isohexane, heptane, octane, cyclohexane, methylcyclohexane, and the like. In addition, these solvents may be used alone or in combination.

The protonic acid to be used in the treatment of compound (4) is not particularly limited, and examples thereof include hydrochloric acid, sulfuric acid, nitric acid, hydrobromic acid, hydroiodic acid, and the like.

The amount of the protonic acid to be used for the production of compound (3) is desirably an equimolecular amount (1 - 1.01 mol, preferably 1 mol) per 1 mol of compound (4). When not less than 1 mol of protonic acid is used, the organic phase is preferably washed with water until the pH of the aqueous phase after washing with water becomes not less than 3, so that the protonic acid used will not remain in the organic phase after the treatment. When the pH of the aqueous phase is less than 3, it is feared that the protonic acid salt used remains in the organic phase, and a protonic acid salt of the A is generated in the subsequent reaction with the A and remains in the composition of the present invention to be a catalyst poison during polymerization.

In the production method of the present invention, the solution of compound (3) prepared as mentioned above can be used as it is for the reaction with the A.

As the A to be used in the production method of the present invention, the aforementioned 5- or 6-membered monocyclic nitrogen-containing aromatic heterocyclic compound having a total carbon number of not less than 25 (preferably not less than 30, more preferably not less than 35) can be mentioned. Specific examples of A include 2,5-dinonadecylpyridine, 2,6-dinonadecylpyridine, 2-nonadecyl-5-octadecylpyridine, 2-nonadecyl-4-octadecyloxypyridine, 2-nonadecyl-6-octadecyloxypyridine, 4-nonadecyl-1-octadecylimidazole, 5-nonadecyl-1-octadecylimidazole, 2-nonadecyl-1-octadecylimidazole, and the like.

Among them, compound (1) obtained by reacting compound (3) and A having a total carbon number of not less than 25 and having two or more (preferably two) C₉₋₃₀ alkyl groups (preferably C₁₄₋₃₀ alkyl groups) or C₉₋₃₀ alkoxy groups (preferably C₁₄₋₃₀ alkoxy groups) is also soluble in aliphatic hydrocarbon solvents.

The reaction temperature and the time in the production method of the present invention are not particularly limited. The reaction temperature is generally 0°C to 40°C, preferably 10°C to 35°C, more preferably room temperature (15°C to 30°C), and the time is not less than 10 min.

After completion of the reaction of compound (3) and the aforementioned A, the reaction solution is dehydrated with a desiccant such as anhydrous sodium sulfate, anhydrous magnesium sulfate, or the like, and then the solvent is removed, whereby compound (1) can be obtained.

In another method, after completion of the reaction of compound (3) and the aforementioned A, a part of the reaction solvent is evaporated or solvent dilution or solvent evaporation (solvent substitution) is performed once or multiple times, whereby a solution of compound (1) can be obtained.

A preferred embodiment of the aforementioned compound (3) is similar to the preferred embodiment of the anionic part (anionic part of compound (1-1) to compound (1-3)) in the aforementioned compound (1).

As a still another method, a solution of compound (1) can also be obtained by preparing in advance a salt of the aforementioned A with protonic acid (e.g., hydrochloride of A), mixing an equimolar amount of the salt and compound (4) in a solvent, and stirring them,

The kinds of protonic acid and solvent, the reaction temperature, the reaction time, and the like in other methods are similar to those in the aforementioned production method of the present invention.

As preferred compound (4), the following compounds can be mentioned.

### [Compound (4-1)]

Compound (4) of the aforementioned formula (4), wherein
R¹, R², R³, and R⁴ are each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 3-phenanthryl group, or a 9-phenanthryl group, each substituted by one or more fluorine atoms or fluoro C₁₋₄ alkyl groups (e.g., trifluoromethyl groups),
M is lithium, sodium, potassium, calcium, magnesium, or barium, and
n is 1 or 2.

### [Compound (4-2)]

Compound (4) of the aforementioned formula (4), wherein
R¹, R², R³ and R⁴ are each independently a phenyl group, a 1-naphthyl group, or a 2-naphthyl group, each substituted by one or more fluorine atoms or trifluoromethyl groups,
M is lithium, sodium, or potassium, and
n is 1.

### [Compound (4-3)]

Compound (4) of the aforementioned formula (4), wherein
R¹, R², R³, and R⁴ are all the same and are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups,
M is lithium or sodium, and
n is 1.

Specific preferred examples of compound (4) include known compounds such as lithium tetrakis(pentafluorophenyl)borate, sodium tetrakis(pentafluorophenyl)borate, lithium tetrakis(nonafluoro[1,1'-biphenyl]-4-yl)borate, lithium tetrakis(heptafluoro-2-naphthyl)borate, lithium [3,5-bis(trifluoromethyl)phenyl]borate, sodium [3,5-bis(trifluoromethyl)phenyl]borate, lithium tetrakis(2,3,4,5,6,7,8-heptafluoro-1-naphthyl)borate, lithium tetrakis(1,3,4,5,6,7,8-heptafluoro-2-naphthyl)borate, sodium tetrakis(2,3,4,5,6,7,8-heptafluoro-1-naphthyl)borate, sodium tetrakis(1,3,4,5,6,7,8-heptafluoro-2-naphthyl)borate, and the like.

The compound of the present invention is soluble in hydrocarbon solvents, and does not contain a compound that could be a catalyst poison such as basic and highly nucleophilic amine compound, protonic acid salt thereof, ether compound with a total carbon number of not more than 7, and the like. Therefore, it is useful as a cocatalyst for the polymerization of olefins and dienes.

The present invention includes a production method of a polymer by polymerizing at least one kind of monomer selected from the group consisting of an olefin and a diene, by using the compound of the present invention as a cocatalyst.

Production of a polymer by using the compound of the present invention as a cocatalyst can be specifically performed according to, for example, the method described in the below-mentioned Experimental Example.

### [Example]

The present invention is specifically explained in detail in the following by referring to Production Examples and Examples; however, the present invention is not limited to those Production Examples and Examples alone. % means mol/mol% for yield and wt% for others unless particularly indicated. The room temperature refers to a temperature of from 15°C to 30°C unless particularly indicated.

For the analysis, the following instrument was used.

¹H-NMR and ¹⁹F-NMR: 400YH (JEOL) manufactured by JEOL Ltd.

Unless particularly indicated, the solvents and reagents used in the following Examples were purchased from distributors such as Sigm-Aldrich, Tokyo Chemical Industry Co., Ltd., FUJIFILM Wako Pure Chemical Corporation, JUNSEI CHEMICAL CO., LTD., KANTO CHEMICAL CO., INC., Combi-Blocks, Inc., and the like. The deuterated solvents used for NMR measurement were purchased from Cambridge Isotope Laboratories.

### [Production Example 1]

### Synthesis of 2,6-bis(nonadecen-1-yl)pyridine

To a mixture of pyridine-2,6-dicarbaldehyde (1.0 g, 7.4 mmol), octadecyltriphenylphosphonium bromide (10 g, 17 mmol), and tetrahydrofuran (100 mL) was added potassium tert-butoxide (2.0 g, 18 mmol) at room temperature. The mixture was stirred at 60°C for 2 hr, and allowed to cool to room temperature. The reaction mixture was carefully added to water, and the mixture was extracted with ethyl acetate. The organic phase was washed with saturated brine solution, dried over anhydrous sodium sulfate, and the solvent was evaporated under reduced pressure. The obtained residue was suspended in diethyl ether, insoluble material was filtered off, and the filtrate was concentrated under reduced pressure. The residue was purified by silica gel column chromatography (n-hexane/ethyl acetate =98/2 to 90/10) to give 2,6-bis(nonadecen-1-yl)pyridine (E/Z mixture; 3.9 g, 86%) .
¹H NMR (CDCl₃) δ: 0.88 (6H, t), 1.20-1.48 (60H, m), 2.56-2.62 (4H, m), 5.82-5.89(1H, m), 6.42-6.49 (2H, m), 7.04 (2H, d), 7.26-7.35 (1H, m), 7.53-7.57 (1H, m).

### [Production Example 2]

### Synthesis of 2,6-di(nonadecyl)pyridine

A mixture of 2,6-bis(nonadecen-1-yl)pyridine (E/Z mixture; 3.5 g, 5.8 mmol) obtained in Production Example 1, 10% Pd/C (containing water (50%); 0.70 g), and tetrahydrofuran (100 mL) was stirred under a hydrogen atmosphere at room temperature and normal pressure for 15 hr. The mixture was filtered, and the filtrate was concentrated under reduced pressure. The obtained residue was purified by silica gel column chromatography (n-hexane/ethyl acetate =95/5) to give 2,6-di(nonadecyl)pyridine (3.0 g, 85%).
¹H NMR (CDCl₃) δ: 0.88 (6H, t), 1.17-1.40 (64H, m), 1.65-1.70 (4H, m), 2.72-2.76 (4H, m), 6.93 (2H, d), 7.48 (1H, t).

### [Production Example 3]

### Synthesis of 2,6-di(nonadecyl)pyridine hydrochloride

To an n-hexane solution (30 mL) of 2,6-di(nonadecyl)pyridine (3.0 g, 4.9 mmol) obtained in Production Example 2 was added 1 M hydrogen chloride-diethyl ether solution (10 mL) at room temperature, and the mixture was stirred for 1 hr. The obtained precipitate was collected by filtration, washed with n-hexane, and dried under reduced pressure to give 2,6-di(nonadecyl)pyridine hydrochloride (3.0 g, 94%) .
¹H NMR (CDCl₃) δ: 0.88 (6H, t), 1.24-1.45 (64H, m), 1.79-1.87 (4H, m), 3.32 (4H, br), 7.41 (2H, d), 8.08 (1H, br).

### [Example 1]

### 2,6-Di(nonadecyl)pyridinium tetrakis(pentafluorophenyl)borate

2,6-Di(nonadecyl)pyridine hydrochloride (0.50 g, 0.77 mmol) obtained in Production Example 3 and lithium tetrakis(pentafluorophenyl)borate mono(diethyl ether) complex (0.59 g, 0.78 mmol) was suspended in dichloromethane (20 mL), and the mixture was stirred at room temperature for 1 hr. The obtained suspension was filtered, and the filtrate was concentration under reduced pressure at 50°C to give 2,6-di(nonadecyl)pyridinium tetrakis(pentafluorophenyl)borate (0.99 g, 99%).
¹H NMR (CDCl₃) δ: 0.85-0.89 (6H, m), 1.23-1.35 (64H, m), 1.72-1.76 (4H, m), 2.94-2.98 (4H, t), 7.57 (2H, d), 8.27 (1H, dd); ¹⁹F NMR (CDCl₃) δ: -133.3 (8F, t), -163.2 (4F, t), -167.7 (8F, t) .

It was confirmed that the compound obtained in Example 1 dissolves in methylcyclohexane at a concentration of 10 wt%.

### [Production Example 4]

### Synthesis of 2-(nonadecen-1-yl)-5-octadecoxypyridine

To a mixture of 5-octadecoxypyridine-2-carbaldehyde (2.0 g, 5.3 mmol), octadecyltriphenylphosphonium bromide (7.0 g, 12 mmol), and tetrahydrofuran (100 mL) was added potassium tert-butoxide (1.4 g, 12 mmol) at room temperature. The mixture was stirred at 60°C for 2 hr, and allowed to cool to room temperature. The reaction mixture was carefully added to water, and the mixture was extracted with ethyl acetate. The organic phase was washed with saturated brine solution, dried over anhydrous sodium sulfate, and concentrated under reduced pressure. The obtained residue was suspended in diethyl ether, insoluble material was filtered off, and the filtrate was concentrated under reduced pressure. The residue was purified by silica gel column chromatography (n-hexane/ethyl acetate=98/2 to 90/10) to give 2-(nonadecen-1-yl)-5-octadecoxypyridine (E/Z mixture; 3.1 g, 95%).
¹H NMR (CDCl₃) δ: 0.87 (6H, t), 1.24-1.50 (60H, m), 1.76-1.80 (2H, m), 2.48-2.54 (2H, m), 3.95-4.00 (2H, m), 7.71-7.78 (1H, m), 6.36-6.40 (1H, m), 7.13-7.18 (2H, m), 8.2-8.27 (1H, m).

### [Production Example 5]

### Synthesis of 2-nonadecyl-5-octadecoxypyridine

A mixture of 2-(nonadecen-1-yl)-5-octadecoxypyridine (E/Z mixture; 2.5 g, 4.1 mmol) obtained in Production Example 4, 10% Pd/C (containing water (50%); 0.70 g), n-hexane (100 mL), and tetrahydrofuran (100 mL) was stirred under a hydrogen atmosphere at room temperature and normal pressure for 15 hr. The reaction mixture was filtered, and the filtrate was concentrated under reduced pressure. The obtained residue was purified by silica gel column chromatography (n-hexane/ethyl acetate=95/5) to give 2-nonadecyl-5-octadecoxypyridine (1.0 g, 40%) .
¹H NMR (CDCl₃) δ: 0.87 (6H, t), 1.17-1.40 (64H, m), 1.42-1.76 (4H, m), 2.67-2.72 (2H, m), 3.95 (1H, t), 7.02 (2H, d), 7.10 (2H, dd), 8.19 (1H, d).

### [Production Example 6]

### Synthesis of 2-nonadecyl-5-octadecoxypyridine hydrochloride

To a mixture of 2-nonadecyl-5-octadecoxypyridine (1.0 g, 1.63 mmol) obtained in Production Example 5 and n-hexane (100 mL) was added 1.0 M hydrogen chloride-diethyl ether (10 mL), and the mixture was stirred for 1 hr. The solvent was evaporated under reduced pressure to give the title compound (0.98 g, 93%).
¹H NMR (CDCl₃) δ: 0.88 (6H, t), 1.24-1.45 (62H, m), 1.78-1.83 (4H, m), 3.14 (2H, t), 4.96 (2H, t), 7.50 (1H, d), 7.73-7.76 (1H, m), 8.20 (1H, d) .

### [Example 2]

### Synthesis of 2-nonadecyl-5-octadecoxypyridinium tetrakis(pentafluorophenyl)borate

2-Nonadecyl-5-octadecoxypyridine hydrochloride (0.25 g, 0.38 mmol) obtained in Production Example 6 and lithium tetrakis(pentafluorophenyl)borate diethyl ether complex (0.29 g, 0.38 mmol) was suspended in cyclohexane (50 mL), and the mixture was stirred at room temperature for 1 hr. The organic phase was washed with brine, dried over anhydrous magnesium sulfate, and concentrated under reduced pressure. The residue was dried under reduced pressure at 45°C to give the title compound (0.45 g, 90%).
¹H NMR (CDCl₃) δ: 0.86-0.90 (6H, m), 2.00-1.44 (62H, m), 1.70-1.86 (4H, m), 2.91 (2H, t), 4.04 (2H, t), 7.66 (1H, d), 7.83 (1H, d), 7.93 (1H, dd);
¹⁹F NMR (CDCl₃) δ: -134.0 (8F, d), -163.4 (4F, t), -167.5 (8F, t) .

It was confirmed that the compound obtained in Example 2 dissolves in methylcyclohexane at a concentration of 10 wt%.

### [Production Example 7]

### Synthesis of 1-octadecylimidazole-2-carbaldehyde

A mixture of 1H-imidazole-2-carbaldehyde (2.0 g, 21 mmol), 1-bromooctadecane (7.5 g, 22 mmol), potassium carbonate (4.5 g, 33 mmol), and N,N-dimethylformamide was stirred at room temperature for 15 hr. The mixture was poured into water, and the mixture was extracted with ethyl acetate. The organic phase was dried over anhydrous sodium sulfate and concentrated under reduced pressure. The residue was purified by silica gel chromatography (n-hexane-ethyl acetate=98/2 to 90/10) to give 1-octadecylimidazole-2-carbaldehyde (6.45 g, 89%).
¹H NMR (CDCl₃) δ: 0.88 (3H, t), 1.24-1.30 (34H, m), 1.75-1.79 (2H, m), 4.36-4.40 (2H, m), 7.15 (1H, s), 7.29 (1H, d), 9.81 (1H, s).

### [Production Example 8]

### Synthesis of 2-(nonadecen-1-yl)-1-octadecylimidazole

To a mixture of 1-octadecylimidazole-2-carbaldehyde (5.0 g, 14 mmol) obtained in Production Example 7, octadecyltriphenylphosphonium bromide (10 g, 16.8 mmol) and tetrahydrofuran (50 mL) was added potassium tert-butoxide (2.0 g, 17.8 mmol) at room temperature. The mixture was stirred at 60°C for 2 hr, and allowed to cool to room temperature. The reaction mixture was carefully added to water, and the mixture was extracted with ethyl acetate. The organic phase was washed with saturated brine solution, dried over anhydrous sodium sulfate, and concentrated under reduced pressure. The obtained residue was suspended in diethyl ether, insoluble material was filtered off, and the filtrate was concentrated under reduced pressure. The residue was purified by silica gel column chromatography (n-hexane/ethyl acetate =98/2 to 90/10) to give 2-(nonadecen-1-yl)-1-octadecylimidazole (E/Z mixture; 7.5 g, 89%).
¹H NMR (CDCl₃) δ: 0.88 (6H, t), 1.11-1.73 (64H, m), 2.20-2.26 (2H, m), 3.85-3.90 (2H, m), 6.11-6.23 (1H, m), 6.67-6.74 (1H, m), 6.81-6.82 (1H, m), 6.98-7.09 (1H, m).

### [Production Example 9]

### Synthesis of 2-nonadecyl-1-octadecylimidazole

A mixture of 2-(nonadecen-1-yl)-1-octadecylimidazole (E/Z mixture; 1.5 g, 2.6 mmol) obtained in Production Example 8, 10% Pd/C (containing water (50%); 0.30 g), and tetrahydrofuran (100 mL) was stirred under a hydrogen atmosphere at room temperature and normal pressure for 15 hr. The mixture was filtered, and the filtrate was concentrated under reduced pressure. The obtained residue was purified by silica gel column chromatography (n-hexane/ethyl acetate=95/5) to give 2-nonadecyl-1-octadecylimidazole (1.0 g, 67%).
¹H NMR (CDCl₃) δ: 0.88 (6H, t), 1.25-1.75 (66H, m), 2.60-2.64 (2H, m), 3.79-3.82 (2H, m), 6.79 (1H, d), 6.93 (1H, d).

### [Production Example 10]

### Synthesis of 2-nonadecyl-1-octadecylimidazole hydrochloride

To a suspension of 2-nonadecyl-1-octadecylimidazole (0.88 g, 1.5 mmol) obtained in Production Example 9 and n-hexane (100 mL) was added 1 M hydrogen chloride-diethyl ether solution (10 mL) at room temperature, and the mixture was stirred for 1 hr. The solvent in the obtained suspension was evaporated under reduced pressure to give 2-nonadecyl-1-octadecylimidazole hydrochloride (0.98 g, 100%).
¹H NMR (CDCl₃) δ: 0.88 (6H, t), 1.25-1.40 (62H, m),1.80-1.88 (4H, m), 3.02-3.07 (2H, t), 3.96-4.00 (2H, t), 6.97 (1H, d), 7.29 (1H, d).

### [Example 3]

### Synthesis of 2-nonadecyl-1-octadecylimidazolium tetrakis(pentafluorophenyl)borate

2-Nonadecyl-1-octadecylimidazole hydrochloride (0.98 g, 1.57 mmol) obtained in Production Example 10 and lithium tetrakis(pentafluorophenyl)borate diethyl ether complex (1.19 g, 1.57 mmol) was suspended in cyclohexane (30 mL), and the mixture was stirred at room temperature for 1 hr. Brine was added thereto, and the organic phase was washed, dried over anhydrous magnesium sulfate, and concentrated under reduced pressure. The concentrate was dried under reduced pressure at 45°C to give 2-nonadecyl-1-octadecylimidazolium tetrakis(pentafluorophenyl)borate (0.82 g, 94%).
¹H NMR (CDCl₃) δ: 0.88 (6H, t), 1.25-1.43 (62H, m), 1.66-1.82 (4H, m), 2.81 (2H, t), 3.94 (2H, t), 6.99 (1H, d), 7.03 (1H, d) ;
¹⁹F NMR (CDCl₃) δ: -133.9 (8F, t), -164.1 (4F, t), -167.9 (8F, t) .

It was confirmed that the compound obtained in Example 3 dissolves in methylcyclohexane at a concentration of 10 wt%.

### [Experimental Example] (Evaluation of polymerization performance)

A general polymerization method using the compound or composition of the present invention as a cocatalyst is shown below.

Into 100 mL autoclave in a glove box were added 1-octene, triisobutylaluminum (TIBA, 0.55 M n-hexane solution) and a solvent (methylcyclohexane (MCH)) to prepare a comonomer solution. A polymerization catalyst (dimethylsilylene(tert-butylamide)-(tetramethylcyclopentadienyl)-titanium (IV)-dichloride (CGC)), triisobutylaluminum (0.55 M n-hexane solution), and a solvent were added to prepare a catalyst solution at a predetermined concentration, and the solution was transferred to a Schlenk flask. The cocatalyst was dissolved in a solvent, and a cocatalyst solution at a predetermined concentration was prepared and transferred to the Schlenk flask. The comonomer solution, the catalyst solution, and the cocatalyst solution were mixed, and adjusted such that the total amount of the solvent and the total amount of triisobutylaluminum would be constant at the time of the reaction. The inside of the autoclave was purged with ethylene gas, the catalyst solution and the cocatalyst solution were successively added to the autoclave, and the ethylene pressure was immediately adjusted to a predetermined pressure, and the mixture was stirred at a predetermined temperature (25°C) for a predetermined time. The reaction mixture was ice-cooled, the ethylene gas was removed, the mixture was poured into methanol (100 mL) containing hydrochloric acid (3 mL), and the mixture was stirred at room temperature for 30 min. The precipitate was collected by filtration and dried under reduced pressure at 60°C to give an ethylene-octene copolymer.

### (Measurement of melting point)

Measurement by the differential scanning calorimetry method (DSC) was performed using DSC6220 instrument (Seiko Instruments Inc.). A sample (polymer) was heated at a rate of 10°C/min from 40°C to 150°C, and the melting point was measured.

The results of the polymerization reaction at 25°C using various cocatalysts are respectively shown in Table 1. As a cocatalyst in Comparative Example 1, N,N-dioctadecylmethylammonium tetrakis(pentafluorophenyl)borate obtained by a method known per se (see, for example, US Patent No. 6121185) was used.

**[Table 1]**

| cocatalyst | catalytic amount (µmol) | solvent | time (min) | yield (g) | activity (kg/mol of Ti·h) | melting point (°C) |
|---|---|---|---|---|---|---|
| Comparative Example 1¹⁾ | 0.5 | MCH | 6 | 0.033 | 5660 | 77.6 |
| Example 1 | 0.5 | MCH | 3 | 0.185 | 7400 | 79.3 |
| Example 2 | 0.5 | MCH | 3 | 1.76 | 70400 | N.D. |

| | | | | | | |
|---|---|---|---|---|---|---|
| Reaction conditions; catalyst: CGC, catalyst:cocatalyst=1:1, TIBA (total amount 3000 pmol), solvent: methylcyclohexane, solvent total amount (40 mL), 1-octene (1 mL), ethylene pressure (8 atm), 25°C ¹⁾ di(octadecyl)methylammonium tetrakis(pentafluorophenyl)borate | | | | | | |

According to Table 1, it was confirmed that Examples 1 and 2 show polymerization activity higher than that of Comparative Example 1.

### [Industrial Applicability]

The compound of the present invention is soluble in hydrocarbon solvents, and does not become a catalyst poison. Thus, it is useful as a cocatalyst for polymerization of olefins and dienes.

This application is based on patent application No. 2020-209071 filed in Japan (filing date: December 17, 2020), the content of which is incorporated in full herein.

## Claims

1. A compound represented by the following formula (1): 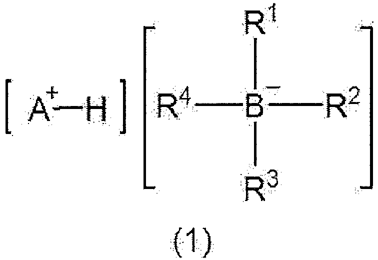
wherein R¹, R², R³ and R⁴ are each independently a C₆₋₁₄ aryl group substituted by one or more fluorine atoms or fluoro C₁₋₄ alkyl groups, and
[A⁺-H] is a cation in which the ring nitrogen atom of a 5- or 6-membered monocyclic nitrogen-containing aromatic heterocyclic compound having a total carbon number of not less than 25 and substituted by the same or different, two or more C₁₋₃₀ alkyl groups or C₁₋₃₀ alkoxy groups is protonated.

2. The compound according to claim 1, wherein R¹, R², R³ and R⁴ are each independently a phenyl group, a 1-naphthyl group, a 2-naphthyl group, a 2-biphenylyl group, a 3-biphenylyl group, a 4-biphenylyl group, a 1-anthryl group, a 2-anthryl group, a 9-anthryl group, a 9-phenanthryl group, or a 3-phenanthryl group, each of which is substituted by one or more fluorine atoms or trifluoromethyl groups.

3. The compound according to claim 1, wherein all of R¹, R², R³ and R⁴ are pentafluorophenyl groups, 2,2',3,3',4',5,5',6,6'-nonafluoro-4-(1,1'-biphenylyl) groups, 2,3,4,5,6,7,8-heptafluoro-1-naphthyl groups, or 1,3,4,5,6,7,8-heptafluoro-2-naphthyl groups.

4. The compound according to any one of claims 1 to 3, wherein
A is a 5- or 6-membered monocyclic bicyclic nitrogen-containing aromatic heterocyclic compound having a total carbon number of not less than 25 and substituted by the same or different two C₉₋₃₀ alkyl groups or C₉₋₃₀ alkoxy groups.

5. The compound according to claim 4, wherein the 5- or 6-membered monocyclic nitrogen-containing aromatic heterocyclic compound is pyridine or imidazole.

6. A cocatalyst for polymerization of at least one kind of monomer selected from the group consisting of an olefin and a diene, consisting of the compound according to any one of claims 1 to 5.

7. A method for producing a polymer, comprising polymerizing at least one kind of monomer selected from the group consisting of an olefin and a diene by using the compound according to any one of claims 1 to 5 as a cocatalyst.
